# EUROPEAN PATENT APPLICATION

(11) **EP 1 435 262 A1**
(43) Date of publication of application: **07.07.2004**
(21) Application number: 03022693.0
(22) Date of filing: 07.10.2003
(51) Int. Cl.: B01J 19/00, G01N 33/543

(54) **Method for manufacturing a biosensor via electrophilic polymer layers**

(30) Priority: 07.10.2002 US 416236 P
(71) Applicant: Max-Planck-Gesellschaft zur Förderung der Wissenschaften e.V., 80539 München (DE)
(72) Inventor: Schiller, Stefan, Dipl.-Chem., 65357 Oestrichwinkel (DE); Kambhampati, Kev, Dr., Alameda, CA 94501 (US); Knoll, Wolfgang, Prof. Dr., 55124 Mainz (DE)
(74) Representative: Weiss, Wolfgang, Dipl.-Chem. Dr.

(57) **Abstract**

The invention relates to a method for the rapid fabrication of biosensors, e.g. nucleic acid microarrays, comprising the deposition of electrophilic polymer layers on carriers comprising suitable substrate surfaces, e.g. commercially available plastic slides, and covalently coupling biomolecules to reactive groups in the electrophilic polymer layer.

## Description

The invention relates to a method for the rapid fabrication of coated carriers, particularly biosensors, e.g. nucleic acid microarrays, comprising the deposition of electrophilic polymer layers on carriers comprising suitable substrate surfaces, e.g. commercially available plastic slides or nanoparticles and covalently coupling biomaterials to reactive groups in the electrophilic polymer layer.

The pharmaceutical and biotech industry's race for discovering potent drugs has increased the demand for a wide range of analytical tools that bridge the gap between our understanding of the genome and direct (proteins) and indirect (lipids and carbohydrates) gene products. Currently, numerous different formats of biochips are required to fully exploit the intricacies of nucleic acid (functional genomics), protein (proteomics)(1), lipid (lipomics)(2) and carbohydrate (glycomics)(3) interactions. Biochips based on in-situ synthesis (4) and various kinds of drop-on-demand spotting techniques (5) have been reported so far on a number of chemically functionalized substrates ranging from glass, gold to membranes (6). These fabrication processes are quite time consuming, require skilled labor and are cost intensive. In addition, most of these biochips are suitable only for DNA analysis, and only in certain situations, for antibodies and proteins. The above limitations of conventional microarrays slows down the drug discovery process and requires the need for numerous kinds of biochip systems from different vendors, whose integration into the research pipeline becomes quite cumbersome.

To address the above challenges, it is important to carefully design the biochip-biomolecule interface, that holds the key to the stability, orientation and functional properties of the immobilized biomolecules. It is also important to keep the cost of biochip fabrication low by limiting the number of production steps and by using substrates whose properties can be readily tuned. Here, we report on the use of commercially available slides, e.g. plastic substrates, as a viable alternative to the existing microarray platforms. These substrates are cheap and by using a simple plasma discharge technique, numerous types of reactive surfaces can be generated. Earlier, the concept of spotting DNA on aminated polypropylene substrates using plasma discharge was introduced (7). One of the limitations of this approach is its dependence on electrostatic interaction to bind probe molecules, a concept that is not applicable to most carbohydrates, lipids, proteins and PNA biomolecules. The above limitations can be overcome by modifying slides with a pulsed plasma deposited carboxylic acid anhydride reactive surface. Plasma techniques are advantageous since they are very reliable and robust (no failure observed within a test of over 40 batches of chips that used to characterize hybridization interactions). The resulting biosensors have tunable properties (e.g. film thickness, swelling, density and accessability of reactive groups), their surface properties allow for an efficient ink-jet delivery and a handling under atmospheric conditions is possible (no clean room required). Further, the plasma process delivers sterilized biochips ready to be modified under germfree conditions and requires an ultra short fabrication time (less than 90 minutes).

Thus, the present invention relates to a method for manufacturing a coated carrier comprising the steps:
(a) providing a carrier comprising a substrate surface,
(b) generating an electrophilic polymer layer on said substrate by plasma polymerization, and
(c) covalently coupling a biomaterial to said electrophilic polymer layer.

Further, the present invention relates to a biosensor comprising
(a) a carrier comprising a substrate surface,
(b) an electrophilic polymer layer on said substrate and
(c) a biomaterial covalently coupled to said electrophilic polymer layer.

The carrier is preferably a carrier suitable for use as a microarray in diagnostic applications. The substrate surface may be a glass, metal, semimetal, metal or semimetal oxide, ceramic, or plastic surface or any combination thereof. Especially preferred is a plastice substrate surface. For example, the carrier may be a commercially available plastic slide, e.g. having dimensions from about 10mm² to about 100cm². Further, the carrier may be a nanoparticle.

The plastic substrate surface may be selected from the group consisting of olefinic polymers and copolymers, styrenic polymers and copolymers, acrylic polymers and copolymers, polyethylenglycol polymers, polyurethane and polyurea polymers and copolymers, polyamides, polyimides, polyesters, polyetherketones, polycarbonates, polyethers, polyvinyl polymers and copolymers, polyphenylenes and any combination thereof. Preferred examples are polyethylene polymers and copolymers, polypropylene polymers and copolymers, polyacrylate, poly(methacrylate), polyacrylamide, polyacrylonitrile polymers and copolymers, poly(ethylene terephthalate), polytetrafluorethylene and other halogenated polymers, polyisoprene, poly (N-vinylpyrrolidone), poly(vinylacetrate), polyvinyl alcohol) and poly(ethylene-statistical norbornene) (TOPAS®). Especially preferred examples of plastic substrates are polypropylene and poly(butadiene-norbornene) copolymers such as TOPAS®.

The method of the present invention comprises the generation of the electrophilic polymer layer on the substrate surface. The first embodiment of the method utilizes monomers containing the electrophilic group.(The expression "monomer" will be used in this context not only covering the generally know monomers used in polymer chemistry, it will also include all molecules not able to undergo classical polymerization reactions but being able to be polymerized under plasma conditions.) The electrophilic group containing monomers can be derived from known methods used in peptide synthesis, from the area of condensating agents or agents and functional groups used for nucleophilic substitution reactions. Monomers to be subjected to plasma polymerization need to fullfil the requirements of a sufficient vapor pressure and formation of a polymer film under plasma conditions with a good retention of the electrophilic group or functionality: * Examples are anhydrides which might be cyclic or linear, saturated or unsaturated (double bond), symmetric or asymmetric (mixed) (such as cyclic and unsaturated: maleic anhydride), preferably with a double bond (maleic anhydride or acrylic acid anhydride as symmetrical compound or anhydride derivatives formed from acrylic acid and formic acid, Leuch 's anhydrides, acetic acid etc.),
* acid halides, e.g. chlorides like acryloylchloride,
* azides to be used according to the azide method of Curtius (Curtius, T. Ber. Dtsch. Chem. Ges., 1902, 35, 3226),
* other activated groups like active esters such as N-hydroxy succinimide esters, nitrophenyl esters, trichlorophenyl esters etc.
* unsaturated alkoxyacetylenes
* O-acyl-isourea intermediates (preferably prepared in situ from surface carboxy groups with DCC (Dicyclohexylcarbodiimide)), EEDQ etc.) as well as monomers derived from molecules activated by phosphonium and uronium salt-based coupling reagents, such as BOP, PyBrOP, PyAOP, HBTU, and HATU (preferably the activation of the carbonyl group is conducted after the polymer film preparation).
* acylimidazoles can be used after their formation from carbonyl diimidazole (CDI) and the carboxy groupd containing monomer.
* monomers containing groups known to enhance nucleophilic substitution reactions like mesylates, triflates, tosylates, halogenides - especially bromides might be used to create electrophilic residues of monomers.
* condensation Agents (e.g. DCC)
* isocyanates (e.g. allylisocyanate)
* epoxides
* aldehydes
* ketenes
* ketenimines (e.g. diphenylketene p-tolimine derivatives)
* groups capable of forming carbenes or carbocations (especially secondary or tertiary halogenes)
* molecules (monomers) carrying photoactivateable groups such as coumarines, benzofurane, indoles, anglecines, azides, quinones, diazocompounds ketons (e.g benzophenone and other diphenylketones and acetophenones) and diaziridines.

The second embodiment comprises forming plasma layers or films from monomers having activateable groups like carboxy functions or alcoholes. These polymers are activated, e.g. the electrophilic groups are generated in a second step after the plasma polymerization e.g. by chemical treatment, heat and/or irradiation whereby polymers having electrophilic groups, e.g. as mentioned above are obtained.

Preferably, the elcetrophilic polymer layer is obtained from polymerizable activated carboxylic acid derivatives which may be selected from the group and consisting of carboxylic acid anhydrides, carboxylic acid halides, such as chlorides and carboxylic acid active esters. More preferably, the activated carboxylic acid derivative is an anhydride.

The activated carboxylic acid derivative is polymerizable under plasma deposition conditions. Preferably, the carboxylic acid derivative contains at least one olefinic bond. Examples of suitable olefinic carboxylic acids are maleic acid, fumaric acid, acrylic acid, methacrylic acid, cinnamylic acid and isoprenoide based acids . An especially preferred polymerizable activated carboxylic acid derivative is maleic acid anhydride.

The electrophilic polymer layer which is provided on the substrate surface by plasma treatment, allows for the direct immobilization of biomaterials, such as nucleic acids, nucleic acids analogues, peptides, proteins, carbohydrates, lipids, cells etc. and thus, serves as a universal platform for the manufacture of coated carriers, e.g. biosensors. More particularly, the biomolecules may be selected from single or double stranded, linear, branched or cyclic DNA, cDNA, RNA, PNA (Peptide Nucleic Acid), LNA (Locked Nucleic Acid), PSNA (Phosphothioate Nucleic Acid), proteins such as membrane and transmembrane proteins (ATPase, COX, peroxidases, glucose oxidase, nucleases, growth hormon receptors, integrines etc.), cytokines, interferones, antibodies, antibody fragments, synthetic antibody or fragment analogues, glycoproteines, glycolipids, lipids, small molecules (e.g. steroides, heterocycles) such as pesticides, hormones, antibiotics, vaccines, dyes, synthetic receptors or receptor ligands, cells, cell fragments and other molecules or molecular architectures of interest. Further, all recombinant or genetically modified non naturally found versions of proteins, antibodies, nucleic acids, antisense molecules, functional biomembranes, cells, stem cells and tissues, biomaterials for internal body use, or artificial organs, implants prevention of bio fauling, chemcial analysis are suitable. The biomaterials are covalently linked to the pretreated surface via nucleophilic groups, such as amino and/or hydroxy groups. Most preferably, the biomaterial may be a nucleic acid or a nucleic acid analogue, such as a peptide nucleic acid (PNA).

The present invention also allows a location-resolved coupling of different biomolecule species, e.g. nucleic acids or nucleic acid analogues having different sequences to different areas of the carrier, thus providing an array, e.g. a micro- or nanoarray. These arrays are suitable for ultra high throughput applications and spotting techniques including chemical surface modifications.

The present invention also allows a rapid, reliable and cheap manufacture of coated carriers, which may be utilized for several applications, e.g. biosensors, nano and nicrostructures such as nanocircuits and transducers, functional nanomaterials, nano or microelectromechanical systems and devices (NEMS, MEMS), bioelectronics "lab on chip applications", semiconductors etc. Suitable carriers, e.g. commercially available plastic slides or nanoparticles may be modified into functional biosensors or biochips using a two-step process. The first step involves covalent attachment of an electrophilic reactive polymer to an "inert" carrier followed by coupling of the desired biomaterials in the second step. The entire manufacture can be completed within 90 minutes.

In a preferred embodiment of the invention the carrier may be subjected to a preactivation treatment, e.g. continuous wave radio frequency plasma pretreatment, corona discharge, flame, glow discharge plasma, chemical priming and/or ultraviolet light in the presence of ozone before the plasma polymerization step. Preferably, the electrophilic polymer layer is covalently coupled to the substrate surface.

The conditions for the plasma deposition of the electrophilic polymer layer are variable. Depending on the power of the plasma, puls sequence (plasma on time / plasma off time = duty cycle) and/or polymerization time, the properties of the polymer layer can be adjusted in terms of film thickness, concentration of reactive groups, e.g. anhydride groups, degree of crosslinking of the polymer and swelling characteristics over a wide range. For example, the film thickness may vary in the range of from 10 - 100 nm, preferably 20-40 nm. The degree of crosslinking can be adjusted that only the surface is modified and no swelling is observed. This results in polymeric layers which may be two-dimensionally modified with biomaterials. By decreasing the power, a swelling up to 20% may be achieved, allowing the manufacture of polymeric layers which may be three-dimensionally modified with biomaterials.

Further, the present invention shall be explained in more detail by the following figures and examples:

### Figure Captions

- **Figure 1.**: Schematic of the PPP MA (pulsed plasma polymerized maleic anhydride) modified polypropylene chips that were used in immobilizing PNA probes for conducting PNA-DNA hybridization studies. PNA probes are covalently attached to the surface via the 3'-terminal amino groups (a). This process is also accompanied by the hydrolyzation of the unreacted anhydride groups. Subsequent addition of fluorescent labeled DNA targets (b) leads to efficient hybridization results (c).
- **Figure 2.**: Schematic of the various PNA probes and the fluorescent labeled DNA targets that were used during the hybridization studies.
- **Figure 3a.**: PNA-DNA mismatch discrimination studies on plasma polymerized maleic anhydride films deposited on polypropylene substrates.
- **Figure 3b.**: PNA-DNA mismatch discrimination studies on plasma polymerized maleic anhydride films deposited on a gold substrate. Based on the final fluorescence intensity values, good mismatch discrimination between the mismatch 0 (a), mismatch 1 (b) and mismatch 2(c) was possible.

### Example

### 1. Materials and Methods

### 1. Materials

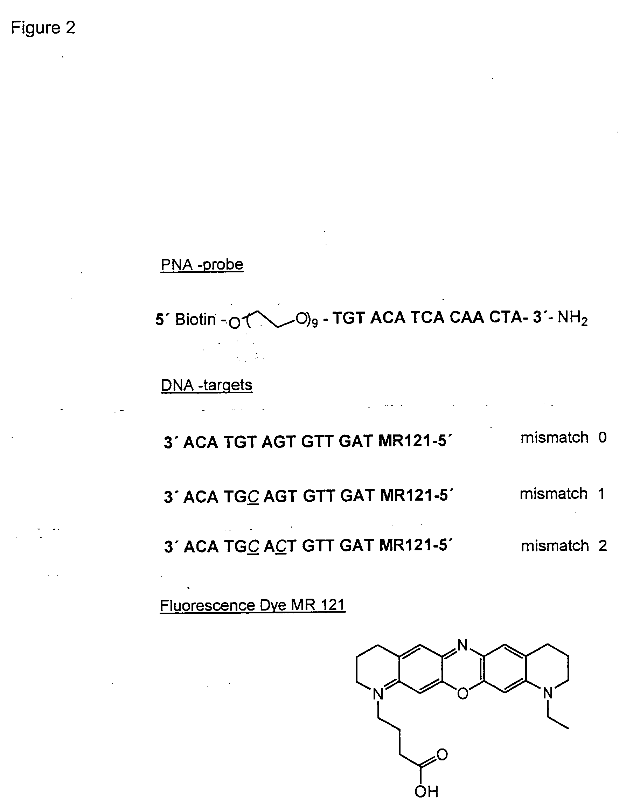

Maleic anhydride was obtained from Fluka (Buchs, Switzerland) while the Polypropylene slides were from Eppendorf pipette containers. Ethanol (chromasolve grade) was used directly without further purification. Ultrapure water was prepared by using a Milli-Q Plus 185 apparatus. The PNA samples were custom sequenced by the Panum Institute in Denmark while MR 121 labeled fluorescent DNA targets were obtained from Boehringer Mannheim, Germany. The MR121 dye can be excited by using a He-Ne laser (633 nm) and emits fluorescence at 670 nm. Probe and target solutions were prepared using a 0.1 M PBS (Sigma) buffer solution. The same buffer was used during the probe immobilization and subsequent hybridization steps. The sequences of the probes and targets as well as the chemical structure of the MR 121 dye is shown in Fig. 2.

### Sample preparation

The polypropylene substrates were cleaned with ethanol and dried in a nitrogen stream and used directly for plasma polymerization without further treatment.

### Plasma Polymerization

The plasma polymerization system was carried out by using a research grade inductively coupled pulsed RF (radio frequency, 13,56 MHz) plasma reactor (10). To ensure reliable starting conditions, the reactor was cleaned before chip fabrication with an argon/oxygen plasma at 0.2 mbar and at a 5 ccm s⁻¹ gas flow rate for each gas (argon/oxygen). The polypropylene samples were placed in the center of the two electrodes parallel to the gas flow direction. The reactor was evacuated to a background pressure of 0.05 mbar or lower, followed by a 30 sec continuous wave plasma (180 W) treatment of the polypropylene slides with the process gas mixture of argon and oxygen described above, directly followed by pulsed plasma polymerization of maleic anhydride. The polymerization of MA was carried out at a working pressure of 0.1 mbar under pulsed plasma conditions using a peak power of 180 W (<P> = 4.4 W). The average power <P> delivered to the system during pulsing was calculated by multiplying the peak power (P_{PEAK}) with the duty cycle (the relation between on and off time) using the following expression:11 <P> = P_{PEAK} {tₒₙ / (tₒₙ + t_{off})}. As standard process parameters, polymerization times of 4 min and a duty cycle of 1 /40 (tₒₙ / t_{off}) in milliseconds/milliseconds) were used and lead to 40 nm ± 3 nm thick plasma polymerized maleic anhydride films.

### Immobilization of PNA Probes on Pulsed Plasma Polymerized Maleic Anhydride Films

Immediately following the plasma polymerization, the plasma treated plastic slides were exposed to a 1 µM PNA probe solution (200 µl) for 30 minutes followed by a rinsing step with MilliQ water. The slides were thereafter, dried under an argon or nitrogen stream and were used directly for experimentation. Slides stored under sealed conditions did not display any drop or loss in hybridization action when they were used after a few days. A 1 µM solution (100 µl) of the various DNA targets (containing various mismatches) was used for the mismatch discrimination hybridization studies. In case of the plastic substrates, hybridization studies were conducted ex-siu. A quartz cuvette (100 µl) was used during SPFS hybridization measurements conducted on gold biochips (12).

### Detection of PNA-DNA Hybridization Interactions

A commercially available biochip fluorescent reader (FLA 2000, Thermo Hybaid) was used for detecting PNA-DNA interactions on plastic substrates. A band-pass filter set as 670 nm was used to detect the final fluorescent signals. Since the SPFS technique works only on metallic substrates, it was used for monitoring PNA-DNA interactions on the gold biochips.

### Detection of radical concentration within the polymer film

ESR measurements to determine the concentration of entrapped radicals were conducted with a Bruker ESP 380 ESR-spectrometer with a rectangular cavity and operated at a frequency of 9.7 GHz. Sample slides with a fifth of the usual width were used and covered with 120 nm plasma polymerized maleic anhydride.

### Measurements of PPP MA polymer film thickness

The PPP MA polymer Film thickness was determined with a Tencor P-10 Surface Profiler.

### 2. Results

We demonstrate the use of plastic chips in monitoring PNA-DNA hybridization interactions using a fluorescence end-point detection scheme. For maleic anhydride films deposited on gold surfaces, a real time Surface Plasmon enhanced Fluorescence Spectroscopy (SPFS) method was used to monitor the above mentioned interactions.

Commercially available polypropylene plastic slides were modified into functional biochips. The entire fabrication step starting from functionalization of plastic substrates with plasma to ready-to-use probe immobilized biochips can be completed within 90 minutes. The fabrication of the polypropylene Pulsed Plasma Polymerized Maleic Anhydride (PPP MA) PNA biochip architectures requires stable chemical linkage between polypropylene and PPP MA in the initial step followed by a linkage between PPP MA and PNA in the following step. The pulsed plasma polymerization of maleic anhydride meets these requirements.

PPP MA films can be covalently linked to the polypropylene slide if reactive species, able to participate in the polymerization reaction of MA, are formed on the polymer surface. To create such a reactive surface, the plastic slides were subjected to continuous wave radio frequency plasma pretreatment before the MA plasma polymerization step. The film chemistry as well as binding characteristics to nucleophiles, may be analyzed by FT-IR, XPS, contact angle measurements and impedance spectroscopy as described in (8). The amount of radicals formed during the plasma process that might be trapped within the film is critical for further biological use of this architecture, and was therefore, determined by ESR spectroscopy. The experiment did not show the existence of radicals down to the detection limit of approximately 10¹⁰ spins. Reactive substrates prepared by this technique are therefore also suitable for cell based studies.

The PPP MA films used in this study were polymerized using a relative peak power of 180 watts resulting in a highly crosslinked polymer, that is accessible for further chemical modification with the PNA probes only at the surface. As a result, the resulting surface geometry of the biochips are comparable with other conventional planar 2D formats. The principal extension of 2D formats to accessible 3D formats is possible because of good swelling properties of PPP MA polymers when there is a lower degree of crosslinking. Such polymer films may as suitable alternatives to polymer brush based DNA-chips (9). The contact angle of the freshly prepared PPP MA layer, a critical parameter during the spotting of probes on the biochip surface, was determined to be 57°. This surface property is ideal for immobilizing probe molecules that are dissolved either in aqueous buffer or organic solvents such as dimethyl sulfoxide.

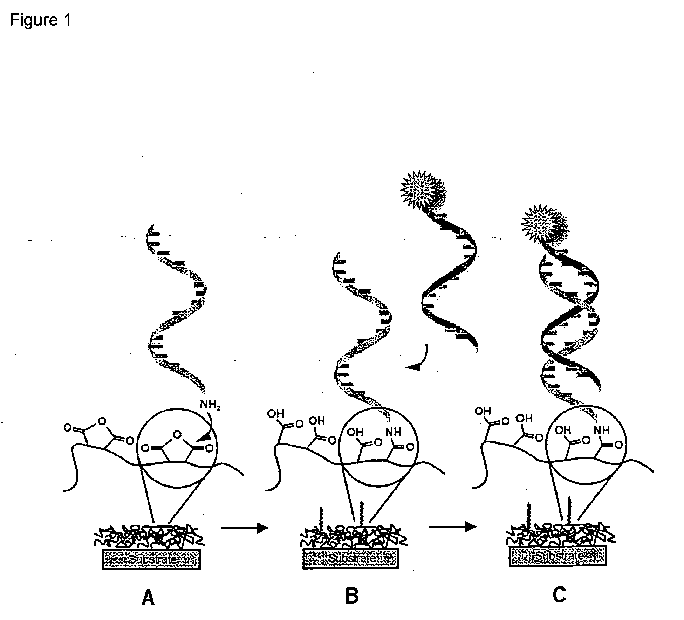

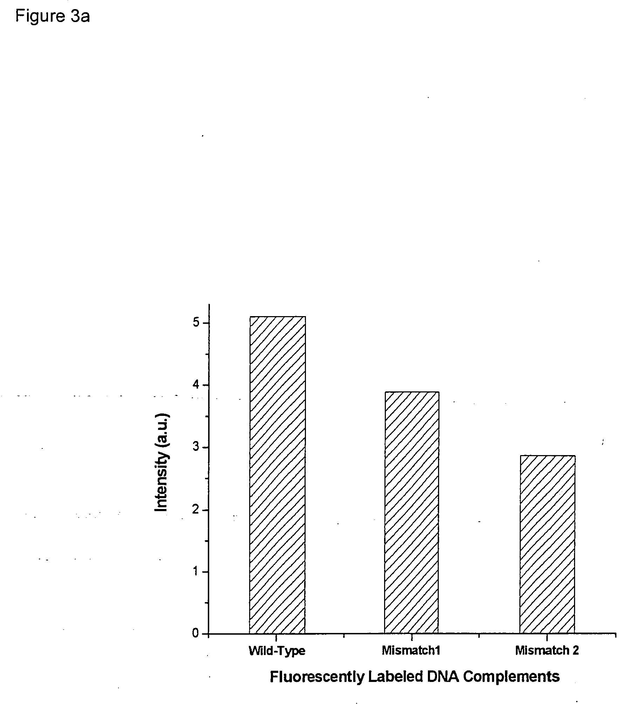

The immobilization of the PNA molecules (Figure 1) on the PPP MA chips was carried out under ordinary room temperature and atmospheric conditions. Expensive clean room infrastructure is not required during this process. PNA probes (1 µM) dissolved in PBS buffer were spotted on to freshly PPP MA modified polypropylene chips using traditional drop-on-demand spotting tools. After a probe incubation period of 30 min, the chips were thoroughly rinsing with Millipore water and subsequently dried under nitrogen. The dried chips were used directly for conducting hybridization experiments. It is worth noting that model experiments involving carboxy terminated self assembled monolayers on gold indicated no electrostatic interactions of the probes with the sensor surface, nor was there any non-specific interaction of the targets on probe free sensor surfaces. These studies clearly indicate that there is specific chemical coupling between the reactive anhydride groups and the PNA probes. The results of PNA-DNA mismatch discrimination studies using the different DNA targets is shown in Fig. 3a. These studies clearly indicate that good mismatch discrimination can be achieved between the wild type (mismatch 0) and the mismatch-1 and 2- targets on plastic chips.

The schematic described in Fig.1 for PNA probe coupling and subsequent hybridization with DNA targets is also applicable to PPP MA films deposited on metallic surfaces. The surface treatment of gold-coated biochips (using small chain alkane thiols) prior to PPP MA film deposition is described in (8). In the case of the gold biochips, SPFS detection technique was used to monitor the interactions of fluorescent labeled DNA targets (containing various mismatches) with the surface immobilized PNA probes, in real-time.

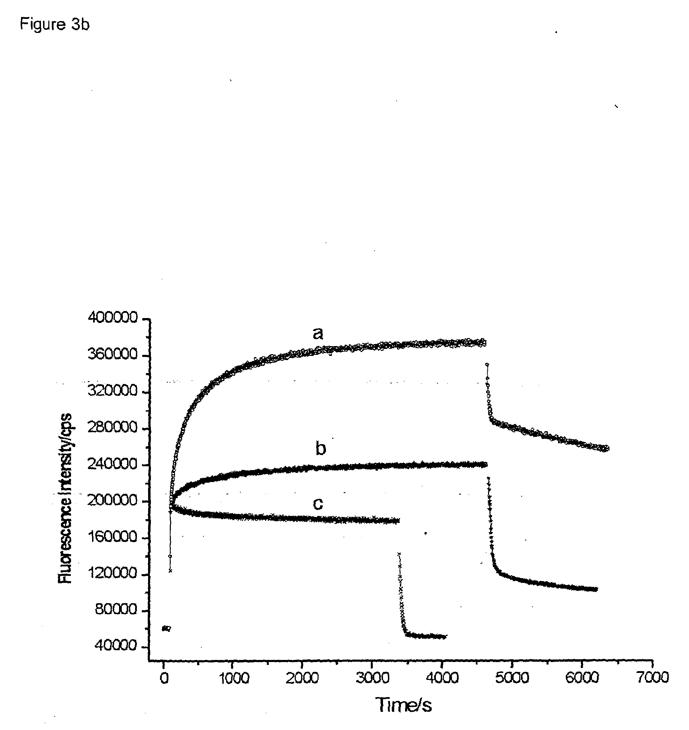

Mismatch discrimination studies similar to those conducted on the plastic chips were also conducted on the gold biochips (Fig.3b). The results are consistent with the earlier analysis, and display good mismatch discrimination (with high signal-to-noise ratio) between the various wild type and mismatched interactions. In the case of the gold chips, the background contribution to the overall signal, as determined from experiments conducted by using fluorescent labeled DNA targets on probe free surfaces, was found to be no more than 5%.

### References

1. Wilkins, M. R., Williams, K. L., Appel, R. D. & Hochstrasser, D. F. (eds.) Proteome Research: New Frontiers in Functional Genomics (Springer Verlag, Berlin Heidelberg New York, 1997).
2. Fitzgerald, D. A. Lipids plus Genomics equals Lipomics. Scientist 16, 42-42 (2002).
3. Hirabayashi, J. & Kasai, K. Glycomics, coming of age! Trends Glycosci. Glycotechnol. 12, 1-5 (2000).
4. Fodor, S. P. A. et al. Light-Directed, Spatially Addressable Parallel Chemical Synthesis. Science 251, 767-773 (1991).
5. Blanchard, A. P., Kaiser, R. J. & Hood, L. E. Synthetic DNA arrays. Biosens. Bioelectron. 11, 687-690 (1996).
6. Southern, E., Mir, K. & Shchepinov, M. Molecular interaction on microarrays. Nat. Genet. 21, 5-9 (1999).
7. Maskos, U. & Southern, E. M. Oligonucleotide hybridizations on glass supports: a novel linker for oligonucleotide synthesis and hybridization properties of oligonucleotides synthesized in situ. Nucleic Acids Res. 20, 1679-1684 (1992).
8. Schiller, S. et al. Chemical Structure and Properties of Plasma-Polymerized Maleic Anhydride Films. Chem. Mater. 14, 235-242 (2002).
9. Rühe, J., Golze, S., Freidank, D., Mohry, S. & Klapproth, H. DNA-chips based on polymer brushes. Abstr. Pap. Am. Chem. Soc. 221, 321-COLL (2001).
10. van Os, M. T., Menges, B., Foerch, R., Vancso, G. J. & Knoll, W. Characterization of plasma-polymerized allylamine using waveguide mode spectroscopy. Chem. Mater. 11, 3252-3257 (1999).
11. Nakajima, K., Bell, A. T. & Shen, M. Plasma Polymerization of Tetrafluoroethylene. J. Polym. Sci., Polym. Chem. Ed. 23, 2627-2637 (1979).
12. Kambhampati, D., Nielsen, P. E. & Knoll, W. Investigating the Kinetics of DNA-DNA and PNA-DNA Interactions Using Surface Plasmon Resonance-Enhanced Fluorescence Spectroscopy. Biosens. Bioelectron. 16, 1109-1118 (2001).

## Claims

1. A method for manufacturing a coated carrier comprising the steps:
(a) providing a carrier comprising a substrate surface,
(b) generating an electrophilic polymer layer on said substrate by plasma polymerization,
(c) covalently coupling a biomolecule to said electrophilic polymer layer.

2. The method of claim 1,
wherein the substrate surface is preactivated.

3. The method of claim 2,
wherein said preactivation comprises a radio frequency plasma pretreatment.

4. The method of any one of the claims 1 to 3,
wherein the electrophilic polymer layer is covalently coupled to the substrate surface.

5. The method according to any one of the claims 1 to 4,
wherein the substrate surface is a plastic surface.

6. The method of claim 5,
wherein said plastic substrate surface is selected from the group consisting of olefinic polymers and copolymers, styrenic polymers and copolymers, acrylic polymers and copolymers, polyethylenglycol polymers, polyurethane and polyurea polymers and copolymers, polyamides, polyimides, polyesters, polyetherketones, polycarbonates, polyethers, polyvinyl polymers and copolymers, polyphenylenes and any combination thereof.

7. The method of claim 6,
wherein said plastic substrate is polypropylene.

8. The method of claim 6,
wherein said plastic substrate is a poly(ethylene-statistical norbornene) copolymer.

9. The method of any one of claims 1-8, wherein the electrophilic polymer layer is generated by plasma polymerization of a polymerizable monomer containing electrophilic groups.

10. The method of any one of claims 1-8, wherein the electrophilic polymer layer is generated by plasma polymerization of a polymerizable monomer containing activateable groups and subsequent activation of said groups in order to obtain electrophilic groups.

11. The method according to any one of the claims 1 to 10,
wherein said electrophilic groups are activated carboxylic acid derivatives which may be selected from the group consisting of carboxylic acid anhydrides, carboxylic acid halides and carboxylic acid active esters.

12. The method of claim 11,
wherein said activated carboxylic acid derivative is a carboxylic acid anhydride.

13. The method according to any one of the claims 1 to 12,
wherein said monomers are selected from carboxylic acid derivatives containing at least one olefinic bond.

14. The method of claim 13,
wherein said olefinic carboxylic acid monomers are selected from maleic acid, fumaric acid, acrylic acid, methacrylic.acid, cinnamylic acid, and isoprenoide based acids.

15. The method of claim 14,
wherein said polymerizable carboxylic acid monomer derivative is maleic acid anhydride.

16. The method according to any one of the claims 1 to 15,
wherein said biomaterial is coupled via an amino or hydroxy group to said electrophilic polymer layer.

17. The method according to any one of the claims 1 to 16,
wherein said biomaterial is selected from the group consisting of nucleic acids, nucleic acid analogues, peptides, proteins, carbohydrates, lipids, cells and cell fragments.

18. The method of claim 17,
wherein said biomaterial is a nucleic acid or a nucleic acid analogue.

19. The method of claim 18,
wherein said biomaterial is a peptide nucleic acid (PNA).

20. The method according to any one of the claims 1 to 19,
wherein step (c) comprises a location-resolved coupling of different biomaterial species to different areas of the carrier.

21. A coated carrier comprising
(a) a carrier comprising a substrate surface,
(b) an electrophilic polymer layer on said substrate and
(c) a biomaterial covalently coupled to said electrophilic polymer layer.

22. The carrier of claim 21,
wherein the substrate surface is plastic.

23. The carrier of claim 21 or 22,
which is a biosensor.

24. The carrier of any one of claims 21-23,
which is an array.
